Europäisches Patentamt

**European Patent Office**

**Office européen des brevets**

(11) Veröffentlichungsnummer: **0 177 776**
A2

# (12) EUROPÄISCHE PATENTANMELDUNG

(21) Anmeldenummer: **85111344.9**

(22) Anmeldetag: **07.09.85**

(51) Int. Cl.⁴: **A 61 F 2/38**

(30) Priorität: **11.09.84 DE 3433264**

(43) Veröffentlichungstag der Anmeldung: **16.04.86**
Patentblatt 86/16

(84) Benannte Vertragsstaaten: **AT BE CH DE FR GB IT LI LU NL SE**

(71) Anmelder: **S + G IMPLANTS GMBH, Grapengiesserstrasse 21, D-2400 Lübeck (DE)**

(72) Erfinder: **Grundel, Hans, Gärtnergasse 4, D-2400 Lübeck (DE)**

(74) Vertreter: **Wilcken, Thomas, Dipl.-Ing. et al, Musterbahn 1, D-2400 Lübeck (DE)**

(54) **Verankerung von Tibiaplateaus.**

(57) Die Verankerung von Tibiaplateaus (6) von Kniegelenk-Endoprothesen besteht darin, daß für jedes Tibiaplateau (6) aus Kunststoff eine metallische, das Plateau aufnehmende Schale (1) vorgesehen ist, deren eine gerade ebene Wandung (2) innen einen in eine waagerechte Ausnehmung (7) des Tibiaplateaus einfassenden waagerechten Vorsprung (5) aufweist, während die sonstige kreisbogenförmige Seitenwand (3) sich von oben bis zum Schalenboden in der Dicke konisch vergrößert. Der Boden und untere Stege, Zapfen oder Dornen (9) sind mit einem metallischen, offenzelligen Belag (4) verbunden, in dessen Zellen Knochengewebe mit anschließender Knochenbildung einwachsen kann, so daß die Schale stets mit dem Schienbein verbunden bleibt, aber das Tibiaplateau auswechselbar ist.

Patentanwälte
Dr. Hugo Wilcken
Dipl.-Ing. Thomas Wilcken
Musterbahn 1 · 2400 Lübeck 1

- 1 -

Anmelder: S + G Implants GmbH,
         Grapengießerstraße 21, 2400 Lübeck


Verankerung von Tibiaplateaus


Die Erfindung bezieht sich auf eine Verankerung von
Tibiaplateaus einer Kniegelenk-Endoprothese im Oberteil
des Schienbeines beidseitig eines von vorn nach hinten
verlaufenden, ebene Seitenflächen aufweisenden Mittelsteges.

Es ist bekannt, Tibiaplateaus mit einer im Oberteil
des Schienbeines zu verankernden Basisfläche mittels
schwalbenschwanzartiger Vorsprünge und Ausnehmungen
durch Einschnappen miteinander zu verbinden (DE-OS 25
50 704) oder Tibiaplateaus durch im Querschnitt
schwalbenschwanzförmige Vorsprünge in entsprechend
profilierte Ausfräsungen des Knochens mit dem Schienbein
zu verbinden (DE-OS 26 08 628). Diese bekannten Verbindungen sind, wie die Praxis gezeigt hat, nicht
stabil genug, da Verankerungen mit glatten Flächen
keine gute Verbindung mit dem Schienbein eingehen und
profilierte Ausfräsungen des Schienbeinknochens schwierig herzustellen sind und ebenfalls nicht stabil genug
erscheinen.

Die Aufgabe der Erfindung besteht darin, eine nicht lösbare Verankerung des Tibiaprothesenteiles im Oberteil des Schienbeines mit einfacher austauschbarer Entnahme der durch Verschleiß verbrauchten Tibiaplateaus zu erreichen.

Diese Aufgabe wird nach der Erfindung dadurch gelöst, daß bei der eingangs erwähnten Verankerung für jedes Tibiaplateau aus Kunststoff eine metallische, auf der Unterseite mit einem metallischen, offenzelligen, die Bildung von Knochen in den Zellen zulassenden Belag versehene Schale vorgesehen ist, die sich mit einer ebenen, innen einen waagerechten Vorsprung aufweisenden Wandung an den Mittelsteg anschließt und deren bogenförmige Seitenwandung sich innen von der Oberkante zum Schalenboden konisch erweitert, und daß das in die Schale einpassende, mit dem Umfang aus der Schale herausragende Tibiaplateau mit einer dem waagerechten Vorsprung zugekehrten Ausnehmung versehen ist.

Durch diese Lösung wird die das Tibiaplateau aufnehmende Schale infolge des auf der Unterseite. vorgesehenen, offenzelligen Belages durch Einwachsen von Knochengewebe mit anschließender Knochenbildung eine unlösbare Verbindung mit der entsprechend vorbereiteten Oberfläche des Schienbeins eingehen. Andererseits kann das Tibiaplateau durch den Eingriff des T-förmigen Vorsprunges in die entsprechende Ausnehmung des Plateaus und durch das Konusprofil der Schalenseitenwandung durch Festklemmen fest in der Schale gehalten, aber durch Anheben des außen liegenden Plateauteiles z.B. mittels eines spitzen Gegenstandes aus der Schale herausgelöst werden.

Die Erfindung wird nachstehend anhand der Zeichnung mit weiteren vorteilhaften Merkmalen erläutert, in der Beispiele dargestellt sind. Es zeigen:

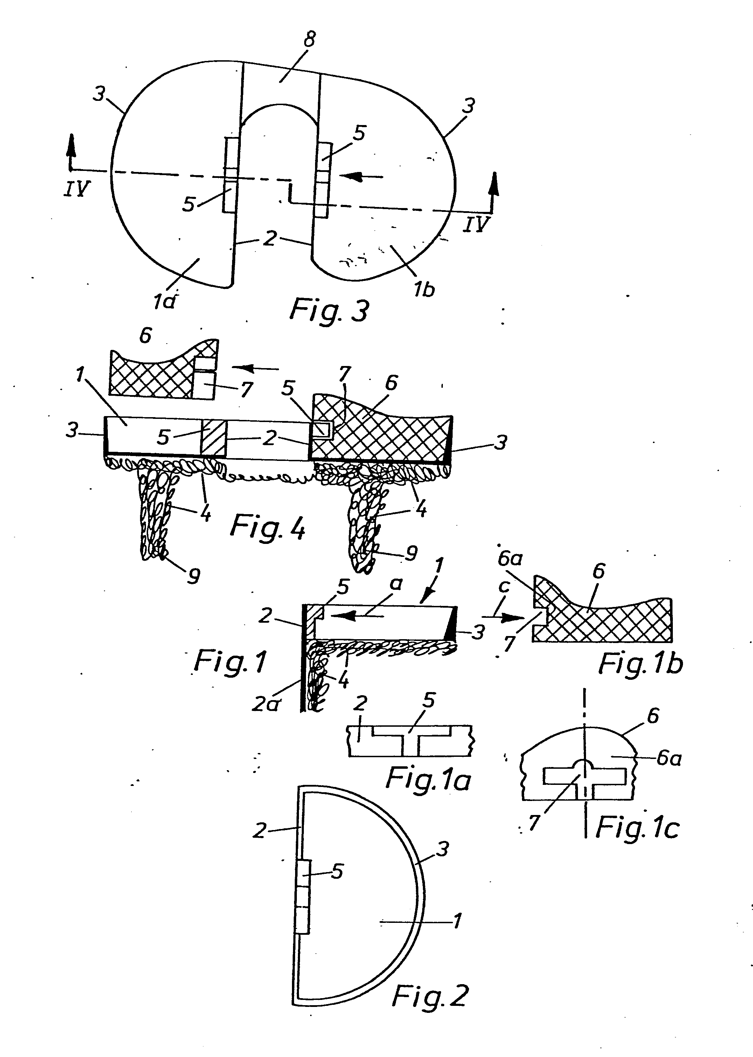

Figur 1    einen senkrechten Querschnitt durch eine
           Verankerung eines Tibiateiles,
Figur 1a   eine Teilansicht in Richtung des Pfeiles a
           der Figur 1,
Figur 1b   einen Querschnitt für das in die Verankerung
           nach Figur 1 passende Tibiaplateau,
Figur 1c   eine Teilansicht in Richtung des Pfeiles c
           der Figur 1b,
Figur 2    eine Aufsicht auf Figur 1,
Figur 3    eine Aufsicht auf zwei starr miteinander
           verbundene Verankerungen der Figur 1,
Figur 4    einen Schnitt nach Linie IV-IV der Figur 3
           mit einem getrennt dargestellten und einem
           in eine der Verankerungen eingesetzten
           Tibiaplateau.

Die Verankerung von Tibiaplateaus von Kniegelenk-Endoprothesen besteht aus einer metallischen Schale 1 (Figur
1) mit einer ebenen, senkrecht zum Schalenboden stehenden
Seitenwand 2 und einer kreisbogenförmigen Seitenwand 3.
An einer Seite eines z.B. aus einem Knochenteil des
Schienbeines bestehenden Mittelsteges (bekannt und daher
nicht dargestellt) mit vorteilhaft parallelen ebenen
Seitenflächen liegt die ebene Schalenwand 2 an. Diese
Wandung 2 ist unten mit einer ebenfalls ebenen Verlängerung 2a versehen, die ebenso wie die Unterseite des
Schalenbodens mit einem metallischen, offenzelligen
Belag 4 versehen ist, in dessen Zellen Knochengewebe
mit anschließender Knochenbildung einwachsen kann, so
daß damit die Schale 1 praktisch unlösbar fest mit dem
Schienbein verbunden wird.

Die ebene Seitenwand 2 ist auf der Schaleninnenseite mit einem waagerechten, vorteilhaft T-förmigen Vorsprung 5 versehen, dessen waagerechte Flansche (Figur 1a) mit der Schalenoberkante abschließen und dessen Steg nach unten gerichtet ist. Die bogenförmige Seitenwand 3 vergrößert sich in der Dicke von oben nach unten konusförmig, wie Figur 1 zeigt.

Die Schale 1 dient zur Aufnahme eines mit oberer Gleitfläche für Kufen eines Femurteiles versehenen Tibiaplateaus 6 aus Kunststoff, welches im Umriß dem Umriß der Schaleninnenfläche angepaßt ist. Dieses Plateau 1b ist auf der ebenen Seitenwand 6a mit einer T-förmigen Ausnehmung 7 versehen, die dem Vorsprung 5 angepaßt ist.

Zur Verbindung des Tibiaplateaus mit der Schale 1 wird es in einer Winkellage in die Schale 1 eingeführt, so daß der T-förmige Vorsprung 5 in die T-förmige Ausnehmung 7 des Tibiaplateaus einfaßt. Sodann wird das Tibiaplateau 6 durch Druck von oben in die Schale 1 hineingedrückt, wobei durch die Konuswandung 3 eine innere Spannung auf das Plateau 6 ausgeübt wird, durch die das Plateau eindeutig fest mit der Schale 1 verbunden ist. Bei einem eintretenden Verschleiß des Tibiaplateaus 6 wird am aus der Schale 1 herausragenden, der Wandung 2 gegenüberliegenden Plateauteil die Spitze eines Gerätes angesetzt, und durch Druck nach oben kann dann das Plateau in eine Winkellage zum Schalenboden gekippt werden, womit auch der Eingriff des T-förmigen Vorsprunges 5 mit der T-förmigen Ausnehmung 7 lösbar ist. Es kann dann wieder ein neues Plateau in die fest mit dem Schienbein verbundenbleibenden Schale 1 eingeführt werden.

Es ist auch möglich, die beiden beidseitig eines Mittelsteges zu verankernden, im Prinzip spiegelbildlichen

Schalen 1a und 1b nach Figur 3 und 4 miteinander zu einer Einheit zu verbinden, indem die ebenen Schalenwandungen 2 im vorderen Bereich durch eine metallische Brücke 8 starr miteinander verbunden werden, sonst aber die Schalen 1a und 1b so auszubilden sind, wie zu Figur 1 und 2 beschrieben ist. In diesem Fall ist es günstiger, den offenzelligen Bodenbelag 4 mit Verankerungsdornen 9 zu verbinden, die vorteilhaft eine Schräglage zum Schalenboden mit Neigung von vorn nach hinten einnehmen.

Allgemein wird der offenzellige Verankerungsbelag 4 bei hohen Temperaturen mit dem Boden der Schale 1 bzw. der Schalen 1a, 1b durch Schmelzschweißung verbunden.

Anmelder: S + G Implants GmbH,
Grapengießerstraße 21, 2400 Lübeck

Ansprüche

1. Verankerung von Tibiaplateaus einer Kniegelenk-Endoprothese im Oberteil des Schienbeines beidseitig eines von vorn nach hinten verlaufenden, ebene Seiten- flächen aufweisenden Mittelsteges, dadurch gekenn- zeichnet, daß für jedes Tibiaplateau (6) aus Kunststoff eine metallische, auf der Unterseite mit einem metallischen, offenzelligen, die Bildung von Knochen in den Zellen zulassenden Belag (4) versehene Schale (1) vorgesehen ist, die sich mit einer ebenen, innen einen waagerechten Vorsprung aufweisenden Wandung (2) an den Mittelsteg anschließt und deren bogenförmige Seitenwandung (3) sich innen von der Oberkante zum Schalenboden konisch erweitert, und daß das in die Schale einpassende, mit dem Umfang aus der Schale herausragende Tibiaplateau (6) mit einer dem waagerechten Vorsprung (5) zugekehrten Ausnehmungen (7) versehen ist.

2. Verankerung nach Anspruch 1, dadurch gekennzeichnet, daß die waagerechten Flansche des Vorsprunges (5) mit der Oberkante der ebenen Schalenwandung (2) abschließen und der Steg des Vorsprunges nach unten gerichtet ist.

3. Verankerung nach Anspruch 1 oder 2, dadurch gekennzeichnet, daß die ebene Schalenwandung (2) bis unter dem Boden nach unten verlängert ist und auf der nach außen gerichteten Fläche mit einem metallischen, offenzelligen Belag (4) versehen ist.

0177776

4. Verankerung nach Anspruch 1, 2, dadurch gekennzeichnet, daß zwei Schalen (1) spiegelbildlich, mit den ebenen Wandungen (2) einander zugekehrt auf dem Vorderende durch eine Brücke (8) starr miteinander verbunden sind und auf der Unterseite mit winkelig gestellten, metallischen, offenzelligen Zapfen fest verbunden ist.

Fig. 3

Fig. 4

Fig. 1

Fig. 1b

Fig. 1a

Fig. 1c

Fig. 2